# FASCICULE DE BREVET EUROPEEN

(11) **EP 0 909 147 B1**
(45) Date de publication et mention de la délivrance du brevet: **10.11.2004**
(21) Numéro de dépôt: 97927244.0
(22) Date de dépôt: 05.06.1997
(51) Int. Cl.: A61F 2/06

(54) **ENDOPROTHESE POUR BIFURCATION VASCULAIRE**
ENDOPROTHESE FÜR EINE VASKULÄRE BIFURCATION
VASCULAR BIFURCATION ENDOPROSTHESIS

(30) Priorité: 06.06.1996 FR 9607245
(43) Date de publication de la demande: 21.04.1999
(73) Titulaire: Seguin, Jacques, 75009 Paris (FR); Laborde, Jean-Claude, 34000 Montpellier (FR)
(72) Inventeur: Seguin, Jacques, 75009 Paris (FR); Laborde, Jean-Claude, 34000 Montpellier (FR)
(86) Numéro de dépôt international: PCT/FR1997/000999
(87) Numéro de publication internationale: WO 1997/046174

(56) Documents cités:
- EP-A- 0 698 380
- WO-A-95/21592
- WO-A-95/32757
- WO-A-96/29955
- WO-A-97/07752
- FR-A- 2 722 678
- US-A- 5 104 404
- US-A- 5 135 536

## Description

La présente invention concerne un dispositif permettant le traitement de conduits corporels au niveau d'une bifurcation, c'est-à-dire au niveau de la séparation d'un conduit principal en deux conduits secondaires.

Ce dispositif peut être utilisé pour le traitement de bifurcations vasculaires, notamment les bifurcations carotidiennes, fémorales, iliaques, poplitée, rénale, ou coronaires, ou non vasculaires, telles que des bifurcations trachéale ou biliaires, par exemple entre les canaux cholédoque et cystique, ou au niveau de la bifurcation de la voie biliaire principale.

Le traitement peut consister à rétablir le diamètre adéquat de la bifurcation, en cas d'artériosclérose ou de prolifération cellulaire interne, à remédier à une dissection localisée ou non de la paroi du conduit, ou, en cas d'anévrisme, à recréer une bifurcation de diamètre normal tout en excluant la poche anévrismale.

Il est connu de traiter le rétrécissement d'un vaisseau sanguin rectiligne au moyen d'un dispositif tubulaire radialement expansible, couramment dénommé "stent". Ce dispositif est introduit à l'état non expansé dans la lumière interne du vaisseau, notamment par voie percutanée, jusqu'au niveau du rétrécissement. Une fois mis en place, il est expansé de manière à étayer la paroi vasculaire et à rétablir la section adéquate du vaisseau.

Le dispositif peut être constitué en un matériau non élastique, et est alors expansé au moyen d'un ballonnet gonflable, sur lequel il est engagé, ou peut être "auto-expandable", c'est-à-dire constitué en un matériau élastique, s'expansant spontanément lors de son retrait hors d'une gaine qui le maintient à l'état contracté.

Les documents US 4 733 665 et US 4 886 062 sont illustratifs de dispositifs existants et de techniques de pose correspondantes.

Un stent classique ne convient pas parfaitement pour le traitement d'un rétrécissement situé au niveau d'une bifurcation, puisque son engagement à la fois dans le conduit principal et dans l'un des conduits secondaires peut provoquer une occlusion immédiate ou retardée de l'autre conduit secondaire.

Il est connu de renforcer une bifurcation vasculaire au moyen d'un dispositif comprenant deux éléments constitués chacun par enroulement hélicoïdal d'un fil métallique. L'un des deux éléments présente deux parties de diamètre correspondant respectivement au diamètre du vaisseau principal et au diamètre de l'un des vaisseaux secondaires, et est destiné à être engagé d'une part dans ce vaisseau principal et d'autre part dans ce vaisseau secondaire. L'autre élément présente un diamètre correspondant au diamètre de l'autre vaisseau secondaire et est accouplé au premier élément, après mise en place de ce dernier, par engagement d'une ou plusieurs de ses spires dans les spires du premier élément.

Ce matériel permet un renforcement de la bifurcation mais apparaît impropre à traiter un rétrécissement vasculaire ou une lésion occlusive, compte tenu de sa structure et de la faible possibilité d'expansion radiale de ses deux éléments constitutifs.

De plus, la forme du premier élément ne correspond pas à la forme d'une bifurcation, qui présente une zone de transition évasée entre l'extrémité du vaisseau principal et les extrémités des vaisseaux secondaires. Ce matériel ne permet donc pas de parfaitement soutenir cette paroi ni de traiter une dissection au niveau de cette paroi.

En outre, la pose séparée de ces deux éléments semble relativement difficile.

Les documents WO 95/21592, EP-A-0 698 380 et WO 95/32757 décrivent des dispositifs de traitement de bifurcations. Ces dispositifs ne permettent pas de réaliser un traitement adéquat de la zone intermédiaire évasée que comprend une bifurcation.

La présente invention vise à remédier à ces différents inconvénients, en fournissant un dispositif permettant de traiter une pathologie au niveau d'une bifurcation, en soutenant parfaitement la paroi vasculaire et en étant relativement simple à poser.

Le dispositif qu'elle concerne comprend, de manière connue en soi, des segments délimitant des conduits longitudinaux, dont un est destiné à être engagé au travers de la bifurcation et dont un autre est destiné à être engagé dans un conduit secondaire de cette bifurcation.

Selon l'invention, le dispositif comporte les caractéristiques de la revendication 1. appui, à l'état expansé, contre la paroi de ce conduit. Cette expansion permet non seulement de traiter un rétrécissement ou une dissection situé au niveau de ce conduit, mais également d'assurer une parfaite immobilisation du dispositif dans le conduit.

Dans cette position du dispositif, le segment tronconique vient prendre appui contre la paroi du conduit délimitant la zone de transition évasée de la bifurcation, qu'il permet d'étayer parfaitement. Un rétrécissement ou une dissection survenant à cet endroit peut donc être traité grâce à ce dispositif, avec un soutien uniforme de la paroi vasculaire, donc sans risque de lésion de celle-ci.

Grâce à la liaison souple qui les relie, les deux segments s'orientent de manière adéquate l'un par rapport à l'autre lors de leur expansion. Le dispositif présente en outre un caractère unitaire, et est donc relativement facile à implanter.

De préférence, au moins le segment tronconique est recouvert par une paroi lui conférant une étanchéité dans une direction radiale.

Cette paroi permet de piéger entre elle et la paroi du conduit les particules pouvant provenir de la lésion traitée, telles que des particules artérioscléreuses ou des agglomérats cellulaires, et d'éviter ainsi la migration de ces particules dans l'organisme.

De plus, le dispositif peut permettre le traitement d'un anévrisme, en assurant le guidage du liquide au travers de la bifurcation, et en évitant donc que la paroi formant l'anévrisme soit sollicitée.

Le dispositif peut comprendre plusieurs segments secondaires, placés les uns à la suite des autres, pour assurer un étayage supplémentaire de la paroi du conduit secondaire et augmenter, si nécessaire, la force d'ancrage du dispositif dans la bifurcation. Dans le même but, le dispositif peut comprendre, du côté du segment tronconique tourné vers le conduit principal, au moins un segment expansible radialement, présentant, à l'état expansé, une section transversale sensiblement supérieure à la section transversale du conduit principal.

Ces différents segments supplémentaires peuvent être reliés entre eux et aux deux segments précités par des liaisons souples telles qu'indiquées plus haut.

De préférence, la liaison souple entre deux segments consécutifs est constituée par un ou plusieurs ponts de matière reliant les deux extrémités adjacentes de ces deux segments. Ce ou ces ponts sont avantageusement constitués par le même matériau que celui constituant les segments.

Selon une forme de réalisation préférée de l'invention, chaque segment a une structure maillée, les mailles étant allongées dans la direction longitudinale du dispositif et présentant chacune une forme sensiblement hexagonale ; les mailles du segment tronconique ont une largeur qui augmente progressivement dans le sens longitudinal du dispositif, en direction de l'extrémité de ce segment ayant la plus forte section à l'état expansé.

Cette augmentation de la largeur des mailles résulte d'une augmentation de la longueur des bords des 5 mailles disposés longitudinalement et/ou d'une augmentation de l'angle que forment entre eux deux bords en regard d'une même maille.

En outre, le segment tronconique peut avoir un axe non confondu avec l'axe longitudinal du dispositif mais oblique par rapport à cet axe, afin d'être adapté au mieux à l'anatomie de la bifurcation à traiter. Dans ce cas, les largeurs des mailles de ce segment augmentent également progressivement, dans le sens transversal du dispositif, en direction d'une génératrice diamétralement opposée à celle se trouvant dans le prolongement du pont reliant ce segment au segment adjacent.

Le dispositif est réalisé en un métal à mémoire de forme, devenant malléable sans élasticité à une température nettement inférieure à celle du corps humain, pour permettre la rétractation du dispositif sur lui-même, et retrouvant sa forme neutre à une température correspondant sensiblement à celle du corps humain. Ce métal est de préférence l'alliage nickel-titane connu sous la dénomination "NITINOL".

Dans un cas comme dans l'autre, ce matériel comprend, selon l'invention, des moyens permettant de repérer, au travers du corps du patient, l'emplacement longitudinal du segment tronconique, afin que celui-ci puisse être correctement positionné au niveau de la zone évasée de la bifurcation.

Dans le cas où l'expansion de ce même segment n'est pas uniforme par rapport à l'axe du dispositif, le matériel comprend en outre des moyens permettant de repérer, au travers du corps du patient, l'orientation angulaire du dispositif par rapport à la bifurcation, afin que la partie de ce segment ayant la plus forte expansion puisse être placée de manière adéquate par rapport à la bifurcation.

Pour sa bonne compréhension, l'invention est à nouveau décrite ci-dessous en référence au dessin schématique annexé représentant, à titre d'exemple non limitatif, deux formes de réalisation préférées du dispositif qu'elle concerne.
La figure 1 en est une vue en de côté, selon une première forme de réalisation ;
la figure 2 est une vue en perspective de ce dispositif dans un état de contraction radiale, et, avec arrachement partiel, du matériel permettant sa pose ;
la figure 3 est une vue en coupe longitudinale de la bifurcation à traiter ;
les figures 4 à 6 sont des vues de cette bifurcation similaires à la figure 3, au cours de trois étapes successives de mise en place du dispositif ;
la figure 7 est une vue similaire à la figure 3 d'une bifurcation présentant un anévrisme, dans laquelle est placé le dispositif, et
la figure 8 est une vue de côté du dispositif selon une deuxième forme de réalisation.

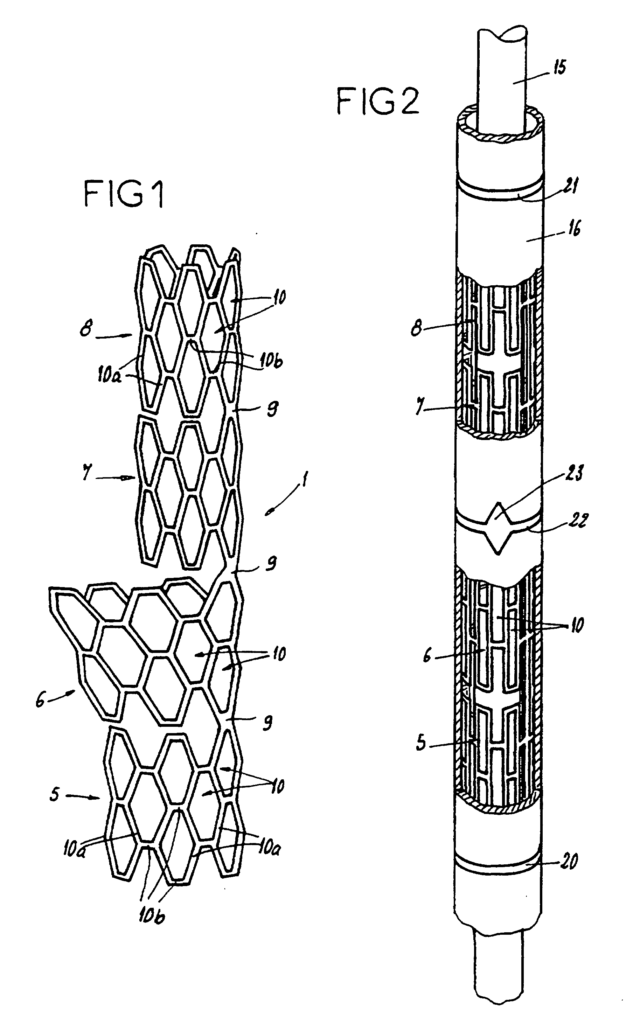

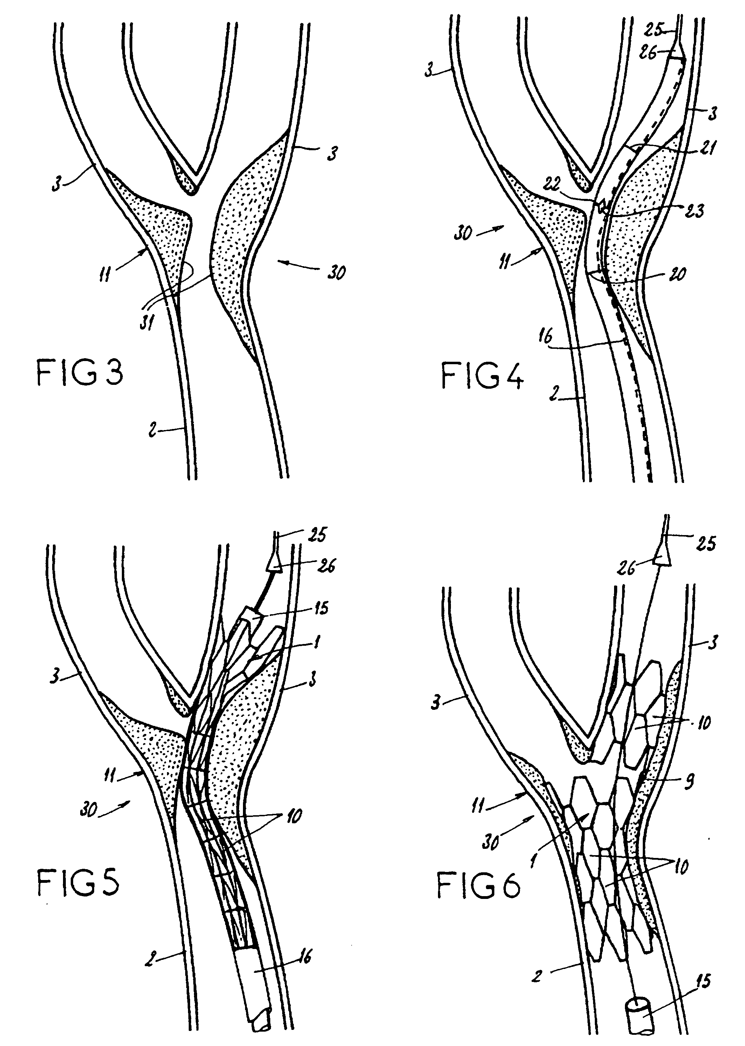

La figure 1 représente un dispositif expansible 1 permettant le traitement de conduits corporels au niveau d'une bifurcation, c'est-à-dire, ainsi que le montre la figure 3, au niveau de la séparation d'un conduit principal 2 en deux conduits secondaires 3.

Le dispositif 1 comprend quatre segments successifs 5,6,7,8 à structure maillée, reliés les uns aux autres par trois ponts de matière 9.

Les mailles 10 de ces segments sont allongées dans la direction longitudinale du dispositif 1 et présentent chacune une forme sensiblement hexagonale.

Le segment 5 présente une forme tubulaire et a un diamètre sensiblement supérieur au diamètre du conduit principal 2.

Le segment 6 présente des mailles 10 dont la largeur augmente progressivement, par rapport à celle des mailles du segment 5, d'une part, dans le sens longitudinal du dispositif 1, en direction de l'extrémité du segment 6 située à l'opposé du segment 5, et, d'autre part, dans le sens transversal du dispositif 1, en direction d'une génératrice diamétralement opposée à celle se trouvant dans le prolongement du pont 9.

Cette augmentation de la largeur des mailles 10 résulte d'une augmentation de la longueur des bords 10a des mailles 10 disposés longitudinalement ainsi que d'une augmentation de l'angle que forment entre eux deux bords 10a en regard.

Ce segment 6 présente ainsi une forme tronconique d'axe oblique par rapport à l'axe longitudinal du dispositif 1. Cette forme correspond à la forme de la bifurcation au niveau de la zone de transition évasée 11 qui sépare l'extrémité du conduit principal 2 des extrémités des conduits secondaires 3.

Les segments 7 et 8 sont identiques entre eux et présentent une forme tubulaire de diamètre sensiblement supérieur au diamètre de l'un des conduits secondaires 3.

Les ponts de matière 9 relient les extrémités adjacentes des segments 5 à 8 et ont une faible largeur, de sorte qu'ils peuvent subir une certaine flexion permettant d'orienter ces segments les uns par rapport aux autres, en particulier le segment 6 par rapport au segment 7.

Le dispositif 1 est réalisé par découpe adéquate d'une tôle en alliage nickel-titane connu sous la dénomination "NITINOL", puis repliage circulaire du flan obtenu et soudure des parties de ce flan venant au voisinage les unes des autres.

Cet alliage est malléable à une température de l'ordre de 10° C, mais peut retrouver sa forme neutre à une température correspondant sensiblement à celle du corps humain.

La figure 2 montre le dispositif 1 dans un état de contraction radiale, obtenu par refroidissement de son matériau constitutif. Lors de cette contraction, les bords 10a pivotent par rapport aux bords transversaux 10b des mailles 10, de sorte que les mailles 10 présentent, dans cet état de contraction, une forme sensiblement rectangulaire.

Grâce à cette contraction, les segments 5 à 8 ont une section transversale inférieure à celle des conduits 2 et 3, et peuvent être introduits dans ceux-ci, ainsi que cela sera décrit plus loin.

Le dispositif 1 est engagé sur une âme centrale de support 15, puis est contracté radialement sur celle-ci. Cette âme 15 comprend une butée axiale, tel qu'un épaulement (non visible sur la figure 2), de diamètre inférieur à celui du dispositif 1 lorsque ce dispositif est expansé, mais supérieur au diamètre de ce dispositif 1 lorsque ce dernier est contracté. Cette butée permet par conséquent l'immobilisation axiale du dispositif 1 sur l'âme 15, lorsque celui-ci est contracté.

Une gaine 16 est ensuite engagée sur le dispositif 1 afin de le contenir dans son état contracté. Cette gaine 16 comporte quatre repères radio-opaques 20,21,22,23 imprimés sur elle, contenant par exemple un composé de baryum. Trois repères 20,21,22 ont une forme annulaire et s'étendent sur l'ensemble de la périphérie de la gaine 16. Ils sont situés respectivement au niveau des extrémités libres des segments 5 et 8 et au niveau du pont 9 séparant les segments 6 et 7. Le quatrième repère 23 est situé à sensiblement mi-distance de la génératrice du segment 6 située dans le prolongement du pont 9 et de la génératrice diamétralement opposée. Il a la forme d'un losange et a une faible épaisseur.

L'âme 15 présente un trou axial longitudinal permettant son engagement sur un fil de guidage 25 (figures 4 à 6). Ce fil 25 peut être engagé, par voie percutanée, dans le conduit 2, au travers de la zone 11 puis dans l'un des conduits 3, au travers desquels il peut coulisser, et comprend un cône 26 de matière synthétique, situé en avant de l'ensemble âme 15 - dispositif 1 - gaine 16.

La bifurcation 30 montrée à la figure 3 présente des excroissances 31 qui créent un rétrécissement de section venant gêner l'écoulement du liquide circulant dans les conduits 2 et 3. Lorsqu'il s'agit d'une bifurcation vasculaire, ces excroissances sont dues par exemple à une artériosclérose ou à une croissance cellulaire.

Le dispositif 1 permet de traiter cette bifurcation, en rétablissant le diamètre adéquat des conduits 2,3 et de la zone évasée 11.

En pratique, ainsi que cela apparaît à la figure 4, l'ensemble âme 15 - dispositif 1 - gaine 16 est engagé sur le fil 25 jusqu'au cône 26. Par son coulissement, ce fil 25 permet l'engagement puis le guidage de cet ensemble dans le conduit 2, la zone 11 puis le conduit 3. Le cône 26 facilite le glissement de l'ensemble et diminue le risque traumatique.

Le repère 22 permet de visualiser, à l'aide d'un appareil radiographique approprié, la position du pont 9 séparant les segments 6 et 7, et donc de visualiser l'emplacement du segment 6, afin que celui-ci puisse être correctement positionné par rapport à la zone évasée 11.

Les repères 20 et 21 permettent de s'assurer que les segments 5 et 8 sont correctement positionnés, respectivement dans le conduit principal 2 et le conduit secondaire 3.

Le repère 23 est, quant à lui, visible en plan ou par sa tranche, selon qu'il est orienté perpendiculairement ou parallèlement au rayon de l'appareil de radiographie. Il permet ainsi de repérer l'orientation angulaire du dispositif 1 par rapport à la bifurcation 30, afin que la partie du segment 6 ayant la plus forte expansion puisse être placée de manière adéquate par rapport à la zone 11.

La gaine 16, qui présente une longueur telle qu'elle débouche au-delà de l'ouverture ayant permis l'introduction de l'ensemble, est ensuite progressivement retirée, comme montré aux figures 5 et 6, pour permettre l'expansion complète du dispositif 1.

Ce dernier est réchauffé par la température corporelle, ce qui permet son expansion.

Après expansion complète du dispositif 1, l'âme 15 et le fil 25 sont retirés.

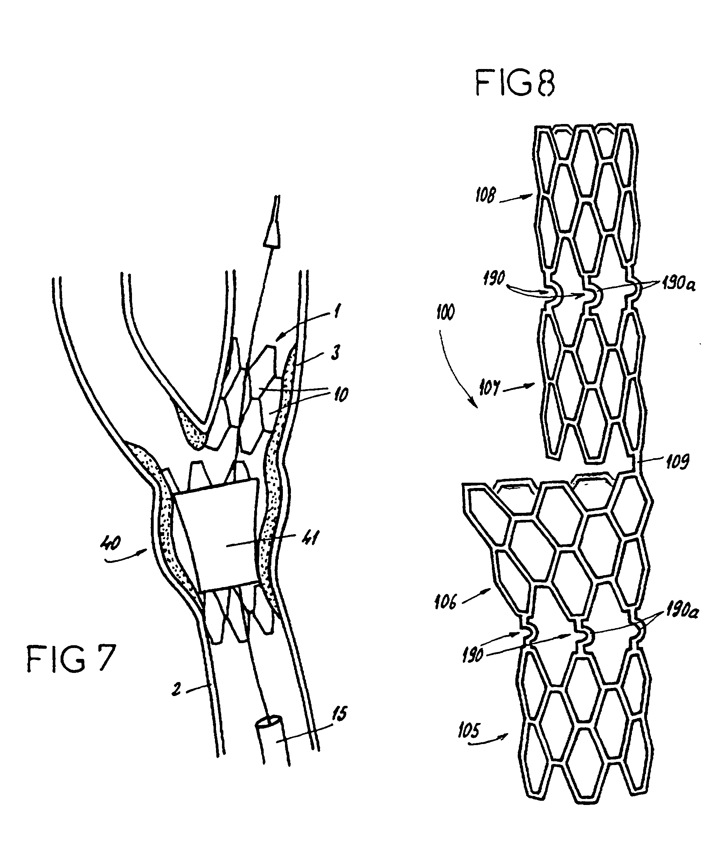

La figure 7 montre que le dispositif 1 peut également servir au traitement d'un anévrisme 40. Le segment 6 et une partie du segment 5 sont alors recouverts d'un film en polyester 41, étanche au liquide circulant dans les conduits 2 et 3, qui est cousu sur eux. Le dispositif guide alors ce liquide au travers de la bifurcation 30, et évite par conséquent que la paroi formant l'anévrisme 40 soit sollicitée.

La figure 8 montre un dispositif 100 selon l'invention, présentant des segments 105,106,107,108 et un pont 109, reliant les segments 106 et 107, de structure similaire à celle des segments 5 à 8 et du pont 9 du dispositif montré à la figure 1.

Dans le dispositif 100, deux segments consécutifs, hormis les segments 106 et 107, sont reliés par six ponts 190 en forme d'oméga. La partie centrale courbe 190a de ces ponts 190 présente une élasticité multi-directionnelle permettant l'orientation longitudinale adéquate des différents segments les uns par rapport aux autres.

Ces ponts 190 ont pour avantage de conférer une continuité longitudinale au dispositif, qui facilite le passage de ce dernier dans une zone à forte courbure et qui élimine la nécessité de procéder à une réduction de cette courbure, toujours dangereuse en cas d'artériosclérose, pour permettre le passage du dispositif.

L'invention fournit ainsi un dispositif permettant le traitement d'une pathologie au niveau d'une bifurcation 30. Ce dispositif présente les nombreux avantages indiqués plus haut, notamment ceux d'assurer un parfait soutien de la paroi vasculaire et d'être relativement simple à poser.

Il va de soi que l'invention n'est pas limitée à la forme de réalisation décrite ci-dessus à titre d'exemple mais qu'elle en embrasse, au contraire, toutes les variantes de réalisation dans le cadre du jeu des revendications annexées.

Ainsi, le dispositif 1,100 peut comprendre plusieurs segments 5,8,105,108 placés les uns à la suite des autres, pour assurer un étayage supplémentaire et augmenter, si nécessaire, le maintien du dispositif dans la bifurcation 30.

L'âme 15 pourrait comprendre un ballonnet gonflable, soit pour réaliser l'expansion du dispositif 1, dans le cas où ce dernier serait en un matériau non élastique, soit pour s'assurer de la totale expansion d'un dispositif 1 auto-expandable, après mise en place de celui-ci.

Les repères 20 à 23 pourraient être imprimés sur l'âme 15 ou directement sur le dispositif 1, notamment sur le pont 9,109, et non sur la gaine 16.

Le segment 6,106 pourrait avoir un axe confondu avec l'axe longitudinal du dispositif, et non oblique par rapport à cet axe, si cela est rendu nécessaire par l'anatomie de la bifurcation à traiter.

En outre, le segment 7,107 pourrait présenter lui-même, à l'état expansé, une forme évasée correspondant à la forme de la zone de raccordement évasée par laquelle, dans certaines bifurcations, les conduits secondaires 3 sont reliés à la zone de transition évasée 11. Ce segment 7,107 présenterait ainsi une forme correspondant précisément à la forme de cette zone de raccordement évasée, dont il assurerait un parfait étayage.

Les ponts 190 pourraient être en nombre supérieur ou inférieur à six entre deux segments consécutifs, et pourraient avoir une forme autre qu'en oméga, permettant leur élasticité multi-directionnelle, et notamment une forme en V ou en W.

## Revendications

1. Dispositif permettant le traitement de conduits corporels au niveau d'une bifurcation, c'est-à-dire au niveau de la séparation d'un conduit principal (2) en deux conduits secondaires (3), comprenant des segments (5 à 8, 105 à 108) délimitant des conduits longitudinaux, dont un est destiné à être engagé au travers de la bifurcation et dont un autre est destiné à être engagé dans un conduit secondaire de cette bifurcation ;
ledit dispositif (1, 100)
étant réalisé en un métal à mémoire de forme, devenant malléable sans élasticité à une température nettement inférieure à celle du corps humain, pour permettre la rétractation du dispositif sur lui-même, et retrouvant sa forme neutre ou "expansée" à une température correspondant sensiblement à celle du corps humain, et il
comprend au moins un segment (7, 8 ; 107, 108) expansible radialement, adapté pour présenter, à l'état expansé, une section transversale sensiblement supérieure à la section transversale de l'un des conduits secondaires (3), de telle sorte que ce segment peut prendre appui, à l'état expansé, contre la paroi de ce conduit secondaire ; **caractérisé**
- **en ce qu'**il comprend un segment (6, 106) dont la structure et le matériau sont adaptés pour que ce segment (6, 106) présente, à l'état expansé, une forme tronconique, cette forme étant telle que ce segment (6, 106) peut prendre appui contre la paroi du conduit délimitant la zone de transition évasée (11) de la bifurcation qui sépare le conduit principal (2) des conduits secondaires (3), et
- **en ce qu'**il comprend une liaison souple (9, 109) reliant le segment (7, 8; 107, 108) expansible radialement et la base la plus large du segment tronconique, ladite liaison souple étant adaptee pour subir une flexion telle qu'elle permet l'orientation de ceux-ci l'un par rapport à l'autre, selon l'orientation du conduit secondaire (3) recevant ledit segment (7, 107) par rapport à ladite zone de transition évasée (11).

2. Dispositif selon la revendication 1, **caractérisé en ce qu'**au moins le segment tronconique (6) adapté pour présenter, à l'état expansé, une forme tronconique est recouvert par une paroi (41) lui conférant une étanchéité dans une direction radiale.

3. Dispositif selon la revendication 1 ou la revendication 2, **caractérisé en ce qu'**il comprend plusieurs segments (7, 8 ; 107, 108) expansibles radialement, placés les uns à la suite des autres et adaptés pour présenter, à l'état expansé, une section transversale sensiblement supérieure à la section transversale de l'un des conduits secondaires (3).

4. Dispositif selon l'une des revendications 1 à 3, **caractérisé en ce qu'**il comprend, du côté du segment tronconique (6, 106) tourné vers le conduit principal (2), au moins un segment (5, 105) expansible radialement, adapté pour présenter, à l'état expansé, une section transversale sensiblement supérieure à la section transversale du conduit principal (2).

5. Dispositif selon l'une des revendications 1 à 4, **caractérisé en ce que** la liaison entre deux segments consécutifs (5 à 8 ; 105 à 108) est constituée par un ou plusieurs ponts de matière (9, 190) reliant les deux extrémités adjacentes de ces deux segments (5 à 8 ; 105 à 108).

6. Dispositif selon la revendication 5, **caractérisé en ce que** le ou les ponts de matière (9) sont constitués par le même matériau que celui constituant les segments (5 à 8 ; 105 à 108).

7. Dispositif selon l'une des revendications 1 à 6, **caractérisé en ce que** chaque segment (5 à 8 ; 105 à 108) a une structure maillée, les mailles (10) étant allongées dans la direction longitudinale du dispositif (1) et présentant chacune une forme sensiblement hexagonale, et **en ce que** les mailles (10) du segment (6 ; 106) adapté pour présenter, à l'état expansé, une forme tronconique, ont une largeur qui augmente progressivement dans le sens longitudinal du dispositif (1), en direction de l'extrémité de ce segment (6 ; 106) ayant la plus forte section à l'état expansé.

8. Dispositif selon l'une des revendications 1 à 7, **caractérisé en ce que** le segment (6 ; 106) adapté pour présenter une forme tronconique après expansion a un axe non confondu avec l'axe longitudinal du dispositif (1) mais oblique par rapport à cet axe, afin d'être adapté au mieux à l'anatomie de la bifurcation (30) à traiter.

9. Dispositif selon l'une des revendications 1 à 8, **caractérisé en ce qu'**il comprend des moyens (20, 21, 22) imprimés sur lui pour permettre de repérer, au travers du corps du patient, l'emplacement longitudinal du segment (6 ; 106) présentant une forme tronconique après expansion.

10. Dispositif selon l'une des revendications 1 à 9, **caractérisé en ce qu'**il comprend des moyens (23) imprimés sur lui permettant de repérer, au travers du corps du patient, son orientation angulaire par rapport à la bifurcation (30).

## Claims

1. A device for treatment of bodily conduits in an area of bifurcation, that is, where a principal conduit (2) separates into two secondary conduits (3), the device comprising segments (5 to 8, 105 to 108) delimiting longitudinal conduits, one of said segments being configured to be engaged through the bifurcation, and another of said segments being configured to be engaged in a secondary conduit of the bifurcation; said device (1, 100) being constructed in a shape memory metal, becoming malleable without elasticity at a temperature that is markedly lower than that of the human body, to allow the device to retract onto itself, and find its neutral or "expanded" form at a temperature substantially corresponding to that of the human body; said device comprising at least one radially expandable segment (7, 8; 107, 108) having, in an expanded state, a cross section substantially greater than the cross section of one of the secondary conduits (3) in such a way that this segment, in an expanded state, may push against the wall of this secondary conduit;
**Characterized in that**
- the device comprises a segment (6, 106) wherein the structure and material are adapted so that this segment (6, 106) has, in an expanded state, a truncated shape, this shape being such that this segment (6, 106) may push against the wall of the conduit delimiting the widened transitional zone (11) of the bifurcation that separates the principal conduit (2) from the secondary conduits (3); and
- the device comprises a flexible link (9, 109) connecting the radially expandable segment (7, 8; 107, 108) and the largest base of the truncated segment, said flexible link being adapted to undergo flexion such that it permits said segments to be oriented in relation to one another on the basis of the orientation of the secondary conduit (3), which receives said segment (7, 107), in relation to said widened transitional zone (11).

2. The device as claimed in claim 1, **characterized in that** at least said truncated segment (6) adapted to have, in an expanded state, a truncated form is covered by a wall (41) which gives said segment impermeability in a radial direction.

3. The device as claimed in claim 1 or claim 2, **characterized in that** the device comprises several radially expandable segments (7, 8; 107, 108) placed one after the other and having, in an expanded state, a cross section that is substantially greater than the cross section of one of the secondary conduits (3).

4. The device as claimed in anyone of claims 1 to 3, **characterized in that** the device comprises, on a side of said truncated segment (6, 106) directed toward the principal conduit (2), at least one radially expandable segment (5, 105) having, in an expanded state, a cross section that is substantially greater than the cross section of the principal conduit (2).

5. The device as claimed in anyone of claims 1 to 4, **characterized in that** the link between two consecutive segments (5 to 8; 105 to 108) is made up of one or more bridge(s) of material (9, 190) connecting adjacent ends of said two segments (5 to 8, 105 to 108).

6. The device as claimed in claim 5, **characterized in that** the bridge(s) (9) of material is or are made of the same material as the material forming the segments (5 to 8, 105 to 108).

7. The device as claimed in anyone of claims 1 to 6, **characterized in that** each of said segments (5 to 8; 105 to 108) has a meshwork structure formed of meshes (10) elongated in a longitudinal direction of the device (1), each mesh (10) having a substantially hexagonal shape, and wherein the meshes (10) of the segment (6, 106) adapted to have, in its expanded state, a truncated shape, have a width which increases progressively in the longitudinal direction of the device (1) in the direction of the end of said segment (6, 106) having the greatest cross section in an expanded state.

8. The device as claimed in anyone of claims 1 to 7, **characterized in that** the segment (6, 106) adapted to have a truncated shape after expansion has an axis that is not coincident with the longitudinal axis of the device (1) but oblique in relation to this axis, in order to be adapted optimally to the anatomy of the bifurcation (30) which is to be treated.

9. The device as claimed in anyone of claims 1 to 8, **characterized in that** the device comprises means (20, 21, 22) printed on itself to allow the longitudinal site of the segment (6; 106) having a truncated form after expansion to be located through the body of the patient.

10. The device as claimed in anyone of claims 1 to 9, **characterized in that** the device comprises means (23) printed on itself to allow its angular orientation with relation to the bifurcation (30) to be located through the body of the patient.

## Patentansprüche

1. Vorrichtung, die das Behandeln von körperlichen Leitungen auf der Ebene einer Vergabelung erlaubt, das heißt, auf der Ebene der Trennung einer Hauptleitung (2) in zwei Nebenleitungen (3), die Segmente (5 bis 8, 105 bis 108) umfasst, die Längsleitungen abgrenzen, von welchen eine dazu bestimmt ist, durch die Vergabelung eingeführt zu werden, und die andere dazu bestimmt ist, in eine Nebenleitung dieser Vergabelung eingeführt zu werden; wobei die Vorrichtung (1, 100) aus einem Metall mit Formgedächtnis hergestellt ist, das ohne Biegsamkeit bei einer Temperatur deutlich unter der des menschlichen Körpers verformbar wird, um das Zusammenziehen der Vorrichtung auf sich selbst zu erlauben, und das seine neutrale oder "ausgedehnte" Form bei einer Temperatur wiederfindet, die im Wesentlichen der des menschlichen Körpers entspricht, wobei die Vorrichtung mindestens ein Segment (7, 8; 107, 108) umfasst, das radial ausdehnbar ist, das im ausgedehnten Zustand einen Querschnitt aufweisen kann, der im Wesentlichen größer ist als der Querschnitt einer der Nebenleitungen (3), so dass sich dieses Segment im ausgedehnten Zustand gegen die Wand dieser Nebenleitung stützen kann;
**dadurch gekennzeichnet,**
- **dass** sie ein Segment (6, 106) umfasst, dessen Struktur und Werkstoff dazu geeignet sind, dass dieses Segment (6, 106) im ausgedehnten Zustand eine kegelstumpfartige Form aufweist, wobei diese Form derart ist, dass sich dieses Segment (6, 106) gegen die Wand der Leitung stützen kann, die die aufgeweitete Übergangszone (11) der Vergabelung abgrenzt, die die Hauptleitung (2) von den Nebenleitungen (3) trennt, und
- dadurch, dass sie eine biegsame Verbindung (9, 109) umfasst, die das radial ausdehnbare Segment (7, 8; 107, 108) und die breiteste Basis des kegelstumpfartigen Segments verbindet, wobei diese biegsame Verbindung eine derartige Biegung erfahren kann, dass sie das Orientieren dieser zueinander nach einer Orientierung der Nebenleitung (3), die das Segment (7, 107) aufnimmt, zu der aufgeweiteten Übergangszone (11) erlaubt.

2. Vorrichtung nach Anspruch 1, **dadurch gekennzeichnet, dass** mindestens das kegelstumpfartige Segment (6), das im ausgedehnten Zustand eine kegelstumpfartige Form aufweisen kann, von einer Wand (41) abgedeckt ist, die ihm eine Abdichtung in eine radiale Richtung verleiht.

3. Vorrichtung nach Anspruch 1 oder Anspruch 2, **dadurch gekennzeichnet, dass** sie mehrere radial ausdehnbare Segmente (7, 8; 107, 108) umfasst, die nacheinander angeordnet sind und im ausgedehnten Zustand einen Querschnitt aufweisen können, der im Wesentlichen größer ist als der Querschnitt einer der Nebenleitungen (3).

4. Vorrichtung nach einem der Ansprüche 1 bis 3, **dadurch gekennzeichnet, dass** sie auf der Seite des kegelstumpfartigen Segments (6, 106), das zu der Hauptleitung (2) gerichtet ist, mindestens ein radial ausdehnbares Segment (5, 105) umfasst, das im ausgedehnten Zustand einen im Wesentlichen größeren Querschnitt aufweisen kann als der Querschnitt der Hauptleitung (2).

5. Vorrichtung nach einem der Ansprüche 1 bis 4, **dadurch gekennzeichnet, dass** die Verbindung zwischen zwei aufeinander folgenden Segmenten (5 bis 8; 105 bis 108) aus einer oder mehreren Stoffbrücken (9, 190) besteht, die die zwei benachbarten Enden dieser zwei Segmente (5 bis 8; 105 bis 108) verbinden.

6. Vorrichtung nach Anspruch 5, **dadurch gekennzeichnet, dass** die Stoffbrücke oder Stoffbrücken (9) aus dem gleichen Werkstoff bestehen wie der, der die Segmente (5 bis 8; 105 bis 108) bildet.

7. Vorrichtung nach einem der Ansprüche 1 bis 6, **dadurch gekennzeichnet, dass** jedes Segment (5 bis 8; 105 bis 108) eine Maschenstruktur hat, wobei die Maschen (10) in die Längsrichtung der Vorrichtung (1) gestreckt sind, und jeweils eine im Wesentlichen sechseckige Form aufweisen, und dadurch, dass die Maschen (10) des Segments (6; 106), das im ausgedehnten Zustand eine kegelstumpfartige Form aufweisen kann, eine Breite haben, die allmählich in die Längsrichtung der Vorrichtung (1) in Richtung des Endes dieses Segments (6; 106), das den stärksten Querschnitt im ausgedehnten Zustand hat, zunimmt.

8. Vorrichtung nach einem der Ansprüche 1 bis 7, **dadurch gekennzeichnet, dass** das Segment (6; 106), das nach dem Ausdehnen eine kegelstumpfartige Form aufweisen kann, eine Achse hat, die nicht mit der Längsachse der Vorrichtung (1) zusammenfällt, sondern zu dieser Achse schräg ist, um besser an die Anatomie der zu behandelnden Vergabelung (30) angepasst zu sein.

9. Vorrichtung nach einem der Ansprüche 1 bis 8, **dadurch gekennzeichnet, dass** sie Mittel (20, 21, 22) umfasst, die auf sie gedruckt sind, um es zu erlauben, durch den Körper des Patienten hindurch die Längslage des Segments (6; 106) zu orten, das nach der Ausdehnung eine kegelstumpfartige Form aufweist.

10. Vorrichtung nach einem der Ansprüche 1 bis 9, **dadurch gekennzeichnet, dass** sie Mittel (23) umfasst, die auf sie gedruckt sind, um es zu erlauben, durch den Körper des Patienten hindurch ihre Winkelausrichtung zu der Vergabelung (30) zu orten.
